# EUROPEAN PATENT APPLICATION

(11) **EP 4 431 006 A1**
(43) Date of publication of application: **18.09.2024**
(21) Application number: 22892537.6
(22) Date of filing: 20.10.2022
(51) Int. Cl.: A61B 3/06

(54) **INFORMATION PROCESSING DEVICE, INFORMATION PROCESSING METHOD, AND COMPUTER PROGRAM**

(30) Priority: 10.11.2021 JP 2021183435
(71) Applicant: MITSUI CHEMICALS, INC., Tokyo 104-0028 (JP)
(72) Inventor: SUZUKI, Kenji, 2990 265 Chiba (JP); MATSUMURA, Shoko, 2990 265 Chiba (JP)
(74) Representative: Mewburn Ellis LLP
(86) International application number: PCT/JP2022/039155
(87) International publication number: WO 2023/085037

(57) **Abstract**

Provided is an information processing device 10 including: a measurement data acquisition unit 101 that acquires gradation characteristics which are a data set obtained by measuring the spectral radiance of an optotype presentation device 20 that presents an optotype to a subject, at plural gradation values of plural primary colors; a stimulus value calculation unit 102 that calculates a stimulus value at each gradation value based on the gradation characteristics; and a function generation unit 103 that fits a predetermined function related to the gradation characteristics to a relationship between the stimulus value and the gradation value.

## Description

### Technical Field

The present disclosure relates to an information processing device, an information processing method, and a computer program.

### Background Art

Techniques used in inspecting a visual function of a subject have been disclosed. For example, Patent Literature 1 discloses a technique related to a visual function inspection system including a visual function inspection means that sequentially presents to a subject optotypes in which a plurality of attention portions have different luminances or stimulus values of color stimuli from each other and executes a visual function inspection on the subject.

Patent Document 1: Japanese Patent Application Laid-Open (JP-A) No. 2019-209047

### SUMMARY OF INVENTION

### Technical Problem

In general, the gradation characteristics (EOTF; Electro-Optical Transfer Function) of an optotype presentation device where an optotype is presented are corrected so as to meet a predetermined standard, but its accuracy varies depending on a product or an individual unit, and in particular, the chromaticity changes in a region where gradation values are small, due to leakage light generated even when the gradation value is set to zero. As a result, even if the gradation value is input to the optotype presentation device according to the color space standard, the optotype is not output with accurate luminance, and the magnitude of an error between the accurate luminance and the display luminance is also unknown. In addition, the chromaticity coordinates of the primary color emitted by the optotype presentation device do not necessarily coincide with the vertex coordinates of the color defined by each standard. As a result, even if the color is calculated based on the chromaticity coordinates of the primary color defined by the color space standard, an error from the actual display color is generated, and the magnitude of the error between the accurate color and the display color is also unknown.

The present disclosure has been made in view of the above aspects, and an object of the disclosure is to provide an information processing device, an information processing method, and a computer program that realize display of the optotype with accurate luminance and chromaticity on the optotype presentation device where the optotype is presented. Solution to Problem

According to one aspect of the present invention, there is provided an information processing device including: a measurement data acquisition unit that acquires gradation characteristics which are a data set obtained by measuring the spectral radiance of the optotype presentation device that presents the optotype to the subject, at a plurality of gradation values of a plurality of primary colors; a stimulus value calculation unit that calculates a stimulus value at each gradation value based on the gradation characteristics; and a function generation unit that fits a predetermined function related to the gradation characteristics to a relationship between the stimulus value and the gradation value.

The function fitted by the function generation unit may include a nonlinear term including a parameter for fitting and a linear term including a parameter for fitting.

The information processing device may further include a gradation value calculation unit that calculates a gradation value corresponding to a display target value in the optotype presentation device based on the function.

The stimulus value calculation unit may calculate tristimulus values of the XYZ color system for each primary color as the stimulus value.

The stimulus value calculation unit may calculate an LMS cone stimulus for each primary color as the stimulus value.

The measurement data acquisition unit may acquire the gradation characteristics at a timing of starting a visual function inspection on the subject.

The measurement data acquisition unit may acquire, in addition to the gradation characteristics, environmental data on an environment at a time point for measuring the gradation characteristics.

The function generation unit may fit the predetermined function to the relationship between the stimulus value and the gradation value according to the environment data.

The measurement data acquisition unit may acquire the gradation characteristics of each of red, green, and blue subpixels that represent the primary color by color mixing.

According to another aspect of the present invention, there is provided an information processing method in which a computer executes processing of: acquiring gradation characteristics which are a data set obtained by measuring the spectral radiance of the optotype presentation device that presents the optotype to the subject, at a plurality of gradation values of a plurality of primary colors; calculating a stimulus value at each gradation value based on the gradation characteristics; and fitting a predetermined function related to the gradation characteristics to a relationship between the stimulus value and the gradation value.

According to another aspect of the present invention, there is provided a computer program that causes a computer to execute processing of acquiring gradation characteristics which are a data set obtained by measuring the spectral radiance of the optotype presentation device that presents the optotype to the subject, at a plurality of gradation values of a plurality of primary colors; calculating a stimulus value at each gradation value based on the gradation characteristics; and fitting a predetermined function related to the gradation characteristics to a relationship between the stimulus value and the gradation value.

### Advantageous Effects of Invention

According to the present disclosure, it is possible to provide an information processing device, an information processing method, and a computer program that realizes display of the optotype with accurate luminance and chromaticity by fitting the predetermined function to the relationship between the actual stimulus value and gradation value of the optotype presentation device and determining the gradation value corresponding to the display target value using the function.

### BRIEF DESCRIPTION OF DRAWINGS

Fig. 1 is a diagram illustrating a schematic configuration of a visual function inspection system according to the present embodiment.
Fig. 2 is a block diagram illustrating a hardware configuration of an information processing device.
Fig. 3 is a block diagram illustrating an example of a functional configuration of the information processing device.
Fig. 4 is a flowchart illustrating a flow of information processing by the information processing device.
Fig. 5 is a diagram illustrating an example of an xy chromaticity diagram of an optotype presentation device.
Fig. 6 is a diagram illustrating an example of dependency of chromaticity coordinates on a gradation value.
Fig. 7 is a diagram illustrating graph indication of fitting of a function to the gradation value dependency of the chromaticity coordinates.
Fig. 8 is a diagram illustrating graph indication of a fitting error of the chromaticity coordinates when the function is fitted to the gradation value dependency of the chromaticity coordinates.
Fig. 9 is a diagram illustrating graph indication of a fitting error of luminance when the function is fitted to the gradation value dependency of the luminance.

### DESCRIPTION OF EMBODIMENTS

Hereinafter, an example of an embodiment of the present disclosure will be described with reference to the drawings. In the drawings, the same or equivalent components and portions are denoted by the same reference numerals. In addition, dimensional ratios in the drawings are exaggerated for convenience of description, and may be different from actual ratios.

### (Background)

First, the background of the embodiment of the present disclosure will be described.

An image display device such as a liquid crystal display, a projector, or a cathode ray tube (CRT) display is used as an optotype presentation device that presents an optotype to inspect a visual function of a subject. In general, as described above, the gradation characteristics of the optotype presentation device where the optotype is presented are corrected so as to meet a predetermined standard. However, the accuracy of the correction varies depending on a product or an individual unit, and in particular, the chromaticity changes in a region where gradation values are small, due to leakage light generated even when the gradation value is set to zero. As a result, even if the gradation value is input to the optotype presentation device according to the color space standard, the optotype is not output with accurate luminance, and the magnitude of an error between the accurate luminance and the display luminance is also unknown.

In addition, the chromaticity coordinates of the primary color emitted by the optotype presentation device do not necessarily coincide with the vertex coordinates of the color defined by each standard. As a result, even if the color is calculated based on the chromaticity coordinates of the primary color defined by the color space standard, an error from the actual display color is generated, and the magnitude of the error between the accurate color and the display color is also unknown. In addition, also in a case in which LED light is used in the optotype presentation device, the same problem occurs due to a phenomenon where the peak wavelength shifts depending on the current value and the chromaticity changes.

Therefore, in view of the above aspects, the present discloser has intensively studied a technique for realizing display of the optotype with accurate luminance and chromaticity. As a result, as described below, the present discloser has devised a technique for realizing display of the optotype with accurate luminance and chromaticity by determining a gradation value corresponding to a display target based on actual gradation characteristics of the optotype presentation device.

### (Schematic configuration)

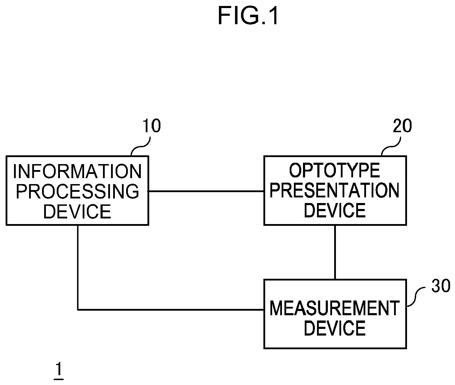

Fig. 1 is a diagram illustrating a schematic configuration of a visual function inspection system according to the present embodiment.

As illustrated in Fig. 1, the visual function inspection system 1 according to the present embodiment includes an information processing device 10, an optotype presentation device 20, and a measurement device 30. In the visual function inspection system 1 according to the present embodiment, it is assumed that the optotype presentation device 20 is a device including red, green, and blue subpixels that represent primary colors by color mixing, such as a liquid crystal display, an organic EL display, or a CRT display. Alternatively, the optotype presentation device 20 may be a projector that presents an image on a wall surface or the like. In addition, the optotype presentation device 20 may present transmitted light or reflected light of an object as an optotype by irradiating the object with illumination such as LED light of a plurality of colors.

The information processing device 10 is a device that determines the gradation value corresponding to the display target based on the actual gradation characteristics of the optotype presentation device 20 and causes the optotype presentation device 20 to present the optotype with the determined gradation value. The information processing device 10 acquires the gradation characteristics of the optotype presentation device 20 from the measurement device 30. Note that the gradation characteristics are a data set obtained by measuring spectral radiance of each of the red, green, and blue subpixels of the optotype presentation device 20 at a plurality of gradation values.

The optotype presentation device 20 is a device that presents an inspection optotype to a subject whose visual function is to be inspected. In the visual function inspection system 1 according to the present embodiment, as described above, the optotype presentation device 20 is a device including the red, green, and blue subpixels that represent primary colors by color mixing, such as a liquid crystal display, an organic EL display, or a CRT display.

In the optotype presentation device 20, the luminance of each of the red, green, and blue subpixels of each pixel constituting the image is controlled by an external image signal, thereby determining the luminance and chromaticity of an image. The image signal corresponds to the gradation value, and the gradation value is calculated and specified by software executed by the information processing device 10.

The measurement device 30 is a device that measures the spectral radiance when the optotype presentation device 20 displays an image with the specified gradation value, and is, for example, a spectral radiance meter. More specifically, the measurement device 30 measures the spectral radiance of each of the red, green, and blue subpixels of the optotype presentation device 20 while changing the gradation value. Since the amount of data is enormous when the measurement is performed for all the gradation values, the measurement device 30 measures the spectral radiance for some representative gradation values.

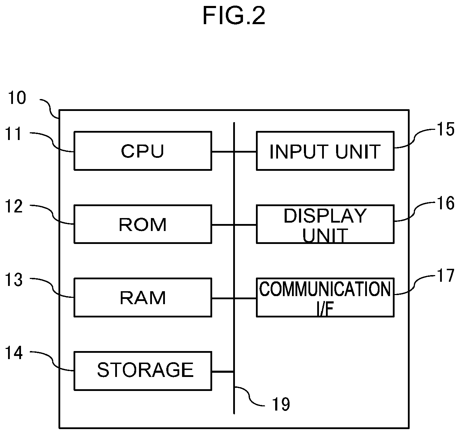

Fig. 2 is a block diagram illustrating a hardware configuration of the information processing device 10.

As illustrated in Fig. 2, the information processing device 10 includes a central processing unit (CPU) 11, a read only memory (ROM) 12, a random access memory (RAM) 13, a storage 14, an input unit 15, a display unit 16, and a communication interface (I/F) 17. The respective components are communicably connected to each other via a bus 19.

The CPU 11 is a central processing unit, and executes various programs and controls each unit. That is, the CPU 11 reads the program from the ROM 12 or the storage 14, and executes the program using the RAM 13 as a work area. The CPU 11 performs control of each of the components and various types of arithmetic processing according to the program recorded in the ROM 12 or the storage 14. In the present embodiment, the ROM 12 or the storage 14 stores an information processing program for realizing display of the optotype with accurate luminance and chromaticity on the optotype presentation device 20.

The ROM 12 stores various programs and various data. The RAM 13 temporarily stores the program or data as a work area. The storage 14 includes a storage device such as a hard disk drive (HDD), a solid state drive (SSD), or a flash memory, and stores various programs including an operating system and various data.

The input unit 15 includes a pointing device such as a mouse and a keyboard, and is used to perform various inputs.

The display unit 16 is, for example, a liquid crystal display, and displays various types of information. The display unit 16 may function as the input unit 15 by adopting a touch panel system.

The communication interface 17 is an interface for communicating with other devices such as the measurement device 30, and for example, uses a standard such as Ethernet (registered trademark), FDDI, or Wi-Fi (registered trademark).

When executing the information processing program, the information processing device 10 realizes various functions using the hardware resources. A functional configuration realized by the information processing device 10 will be described.

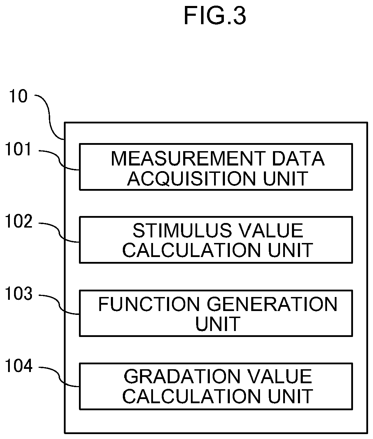

Fig. 3 is a block diagram illustrating an example of a functional configuration of the information processing device 10.

As illustrated in Fig. 3, the information processing device 10 includes a measurement data acquisition unit 101, a stimulus value calculation unit 102, a function generation unit 103, and a gradation value calculation unit 104 as functional components. Each functional component is realized by the CPU 11 reading and executing the information processing program stored in the ROM 12 or the storage 14.

The measurement data acquisition unit 101 acquires the gradation characteristics which are the data set of spectral radiance, measured by the measurement device 30, of each of the red, green, and blue subpixels of the optotype presentation device 20 at the plurality of gradation values. The number of gradation values is not limited to a specific number, but the number of gradation values is determined in consideration of the generation accuracy of the function at the function generation unit 103 described later.

The stimulus value calculation unit 102 calculates tristimulus values for each subpixel with regard to the spectral radiance at each gradation value using the gradation characteristics acquired by the measurement data acquisition unit 101. In the present embodiment, an XYZ color system is used. Therefore, the stimulus value calculation unit 102 calculates tristimulus values X, Y, and Z for each subpixel. The information processing device 10 can calculate the luminance and chromaticity from the tristimulus values by calculating the tristimulus values. Alternatively, the stimulus value calculation unit 102 may calculate an LMS cone stimulus value, or may calculate a rod stimulus value or an intrinsically photosensitive retinal ganglion cell (ipRGC) stimulus value.

The function generation unit 103 fits a predetermined function related to the gradation characteristics to the relationship between the tristimulus values at each gradation value calculated for each subpixel by the stimulus value calculation unit 102 and the gradation value corresponding to the tristimulus values. In the present embodiment, in a case in which the gradation characteristics are gamma characteristics, a function represented by the following formula (1) is fitted to a relationship between the tristimulus values and gradation value.

### [Mathematical Formula 1]

In Formula (1), Tₛₚ (s: X, Y, Z) is a group of tristimulus values X, Y, and Z for each subpixel p (p: R, G, B), nₚ is a gradation value, n_{g} is the maximum gradation value (for example, 255), and γₛₚ, Aₛₚ, and Bₛₚ are parameters for fitting the function. In addition, to further improve the accuracy of the fitting, the function generation unit 103 may generate the function by adding a high-order polynomial of nₚ/n_{g}.

Here, the purpose of calculating the tristimulus values will be described. When controlling the optotype presentation device 20, the simplest method is to represent the gradation characteristics of the luminance Y by a function by assuming that the chromaticity of each light emitting element of the optotype presentation device 20 is constant regardless of the gradation value, that is, the xy chromaticity coordinates are constant. The color of the subpixel of the liquid crystal display is represented by the spectral transmittance of the color filter, and is thus considered not to depend on the light amount of the output. However, when the spectral radiance is actually measured with a spectral radiance meter, the obtained result is that the spectral distribution (the shape of the spectrum of the spectral radiance) changes little by little depending on the gradation value. This is mainly because of the addition of leakage light of another subpixel whose gradation value is set to zero among a large number of pixels captured by the target of the spectral radiance meter. Note that the liquid crystal display has a property that the peak of the emission spectrum is slightly shifted by the heat generation of the current-dependent element and the ambient temperature.

Therefore, it cannot be said that function representation assuming that the xy chromaticity coordinates are constant is appropriate. In addition, since the gradation characteristics of the xy chromaticity coordinates are not a linear function with respect to the spectral radiance, it is difficult to apply an approximation function of an unambiguous form representing the change.

It is thus desirable to select a color stimulus amount represented by a linear function with respect to the spectral radiance to represent the gradation characteristics that take into consideration the change in the spectral distribution of the light emitting element. The tristimulus values of the XYZ color system are one such stimulus amount. In particular, the stimulus value Y corresponds to the luminance. The color-matching functions of the tristimulus values are different from each other, but all the tristimulus values are defined by constant integration of the same form. In a case in which the spectral distribution does not depend on the gradation value, the gradation characteristics of the stimulus values X and Z are obtained by multiplying the gradation characteristics of the stimulus value Y by a constant. Therefore, in a case in which the change in the spectral distribution due to the gradation value is slight, it can be expected that the gradation characteristics of the stimulus values X and Z can be represented by adjusted coefficients based on the same functional form as the stimulus value Y.

The stimulus values X and Z correspond to the xy chromaticity in a simple formula, and thus it is suitable to set the task of changing the luminance while keeping the xy chromaticity of the optotype constant, or vice versa. In a case in which a Landolt ring is used as the optotype, it is possible to perform an experiment of MacAdam's color discrimination ellipse in consideration of the size of the Landolt ring by applying the tristimulus values to the task of changing a difference in xy chromaticity while keeping the luminance of the Landolt ring and the luminance of the background equal and constant.

The LMS cone stimulus value of the LMS color space is also linear with respect to spectral radiance. However, since the absolute amount of the LMS cone stimulus is not defined, it is necessary to perform the operation of associating the stimulus value with the luminance. The LMS cone stimulus value-based gradation characteristics are suitable for experiments where the operation of the silent-substitution method is incorporated to control only the stimulus values of one cone or to control the stimulus values with reference to the r-axis and the b-axis of the MacLeod-Boynton chromaticity diagram. Furthermore, when there are four or more light sources (primary colors), it is possible to perform an experiment for evaluating the relationship between the brightness perception of ipRGC, the discrimination threshold value, and the day blindness under the condition where the luminance or color for photopic vision is constant.

As a measure of the intensity of light, luminance Lᵥ (cd/m²) is defined by the following formula (2) as a photometric amount with respect to spectral radiance Lₑ(λ) (Wsr⁻¹m⁻²nm⁻¹).

### [Mathematical Formula 2]

In Formula (2), V(λ) is the standard spectral luminous efficiency for photopic vision, and Kₘ = 683 (lm/W).

Hereinafter, the color-matching functions ⁻x(λ), ⁻y(λ), and ⁻z(λ) are collectively defined as ⁻xₛ(λ) (s: X, Y, Z), and the tristimulus values X, Y, and Z are collectively defined as Tₛ (s: X, Y, Z). Note that a character where "-" is added above a symbol (for example, X) is represented as ⁻X or the like. The color-matching function ⁻XY(λ) is defined as the same one as V(λ). From T_{Y} = L_{V}, the tristimulus values Tₛ (cd/m²) are represented by the following formula (3).

### [Mathematical Formula 3]

The output of each subpixel p (p: R, G, B) is decomposed into tristimulus values to represent the luminance and color. Since the stimulus value Y coincides with the luminance output of the liquid crystal display, the gamma characteristics can be considered as a basic form as the function representation of the gradation characteristics of the tristimulus values Y, X, and Z. By deploying the gradation characteristics defined by the color space standard to a polynomial formula with a γ power term and an integer power of a normalized gradation value and changing the polynomial formula to the form adding the contribution of leakage light as a constant term, nonlinearity is limited to only one parameter of γ, thus making it easier to use the formula in fitting by a least squares method. The simplest of such forms is Formula (1). Thus, the gradation characteristics of each of the luminance and the color are associated with the spectral radiation luminance for each subpixel.

The gradation value calculation unit 104 calculates a gradation value for presenting the optotype to the optotype presentation device 20 as the display target value, using the function generated by the function generation unit 103. The display target value is, for example, luminance and xy chromaticity coordinates in a case in which the gradation value is calculated using the function representing the tristimulus values, and is the luminance and cone stimulus values or ratio of L, M, and S in a case in which the gradation value is calculated using the function representing the LMS cone stimulus.

With such a configuration, the information processing device 10 can fit the predetermined function to the relationship between the actual stimulus value and gradation value of the optotype presentation device 20 and determine the gradation value corresponding to the display target value using the function. By determining the gradation value corresponding to the display target value, the information processing device 10 can realize display of the optotype with accurate luminance and chromaticity on the optotype presentation device 20.

Next, the operation of the information processing device 10 will be described.

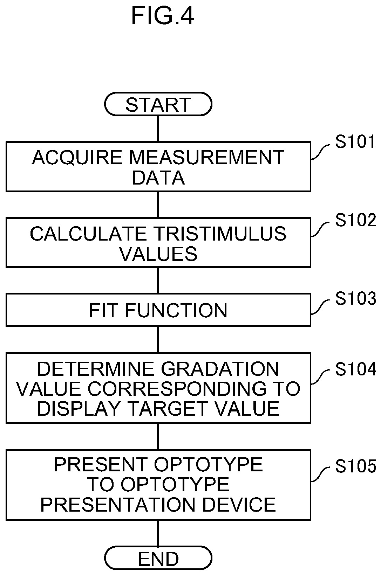

Fig. 4 is a flowchart illustrating a flow of information processing by the information processing device 10. The CPU 11 reads the information processing program from the ROM 12 or the storage 14, deploys the information processing program in the RAM 13, and executes the information processing program, thereby performing information processing. In the following description, it is assumed that the optotype presentation device 20 is a liquid crystal display.

First, in step S101, the CPU 11 acquires measurement data from the measurement device 30. The measurement data here is gradation characteristics which are the data set of the spectral radiance of each of the red, green, and blue subpixels of the optotype presentation device 20 at the plurality of gradation values.

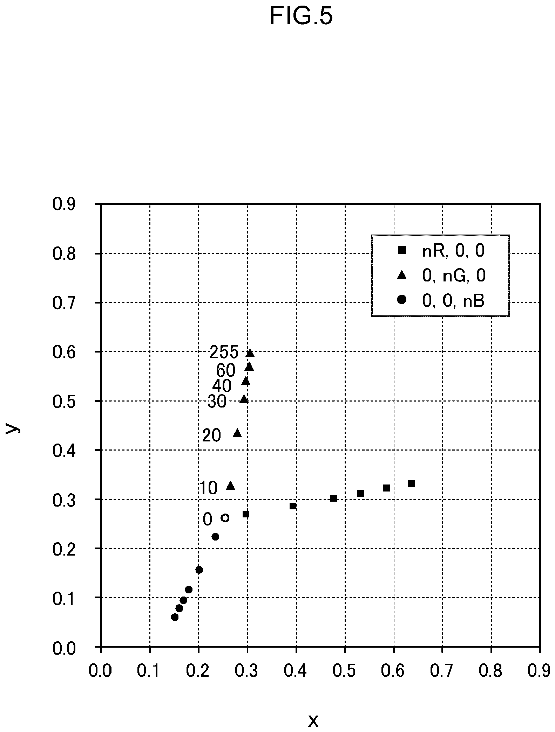

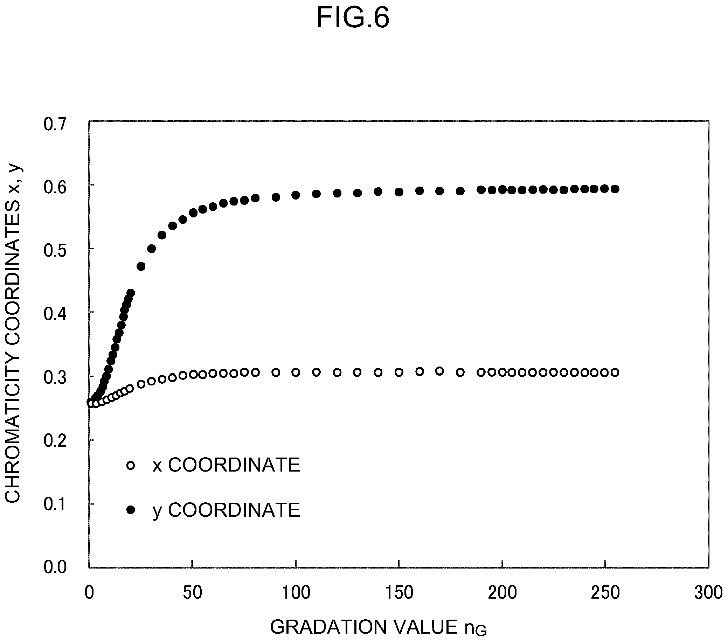

Fig. 5 is a diagram illustrating an example of the xy chromaticity diagram of the optotype presentation device 20. Fig. 6 is a diagram illustrating an example of the gradation value dependency of the xy chromaticity coordinates, and is a diagram illustrating an example of the measurement result by the measurement device 30. Fig. 6 illustrates an example of the gradation value dependency of the chromaticity coordinates in the green subpixel. As illustrated in Fig. 5, in a case in which the gradation value of one subpixel is arbitrary and the gradation value of the other subpixels is zero, the chromaticity coordinates of the image displayed on the screen move as the gradation value decreases. That is, the movement of the chromaticity coordinates cannot be calculated with the gradation characteristics (EOTF) of the optotype presentation device 20 defined by the color space standard. As a result, an error in the presented color of the optotype increases particularly at low gradation values. For example, an error in the xy chromaticity coordinates of 0.001 to 0.005 or more in the order of MacAdam's color discrimination ellipse occurs, which makes the optotype presentation device inappropriate for a color discrimination experiment.

To address this issue, the information processing device 10 acquires the gradation characteristics of the optotype presentation device 20 measured by the measurement device 30 to determine an accurate gradation value corresponding to the display target value. From the gradation characteristics of the optotype presentation device 20 measured by the measurement device 30, the gradation value dependency of the xy chromaticity coordinates as illustrated in Fig. 6 is obtained.

After the measurement data is acquired from the measurement device 30 in step S101, the CPU 11, in step S102, subsequently calculates the tristimulus values for the spectral radiance at each gradation value using the gradation characteristics acquired in step S101. In the present embodiment, an XYZ color system is used. The CPU 11 thus calculates the tristimulus values X, Y, and Z in step S102. By calculating the tristimulus values, the CPU 11 can calculate the luminance and chromaticity from the calculated tristimulus values. Alternatively, the CPU 11 may calculate the LMS cone stimulus value as the stimulus value, or may calculate the rod stimulus or the ipRGC stimulus.

After the tristimulus values are calculated in step S102, the CPU 11, in step S103, subsequently fits the predetermined function to the relationship between the tristimulus values at each gradation value calculated in step S102 and the gradation value corresponding to the tristimulus values. Specifically, the CPU 11 can fit the function to the gradation characteristics of the tristimulus values by adjusting the parameters in Formula (1).

The CPU 11 may hold environmental data that is information on the environment such as the temperature, the atmospheric pressure, and the humidity when the measurement device 30 measures the gradation characteristics, and change the function parameter according to the environment at the time of inspection.

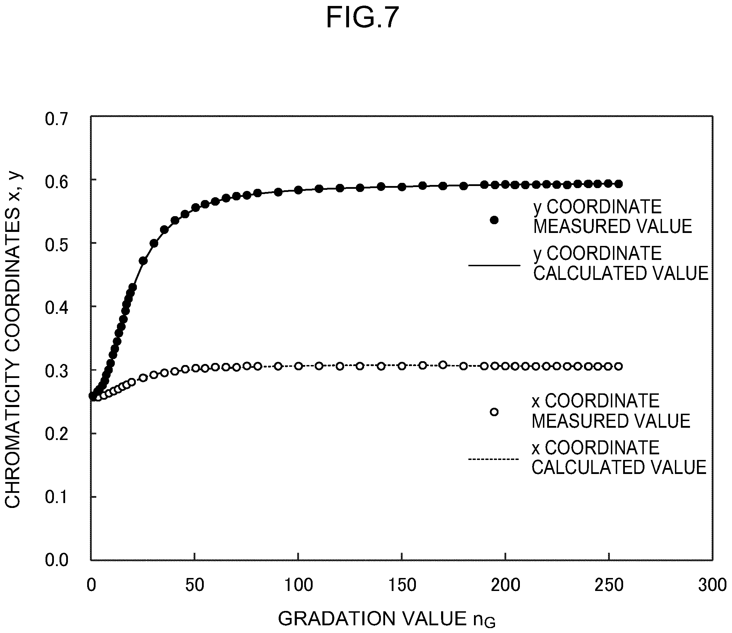

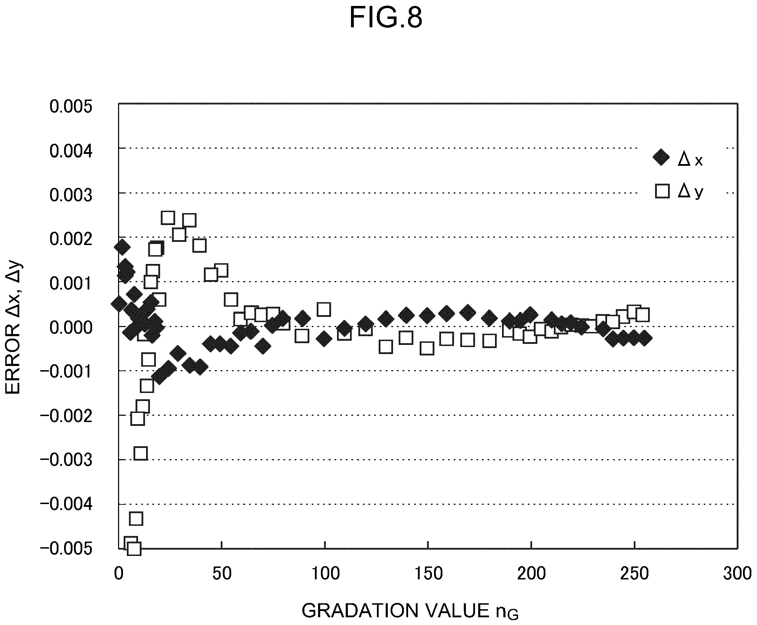

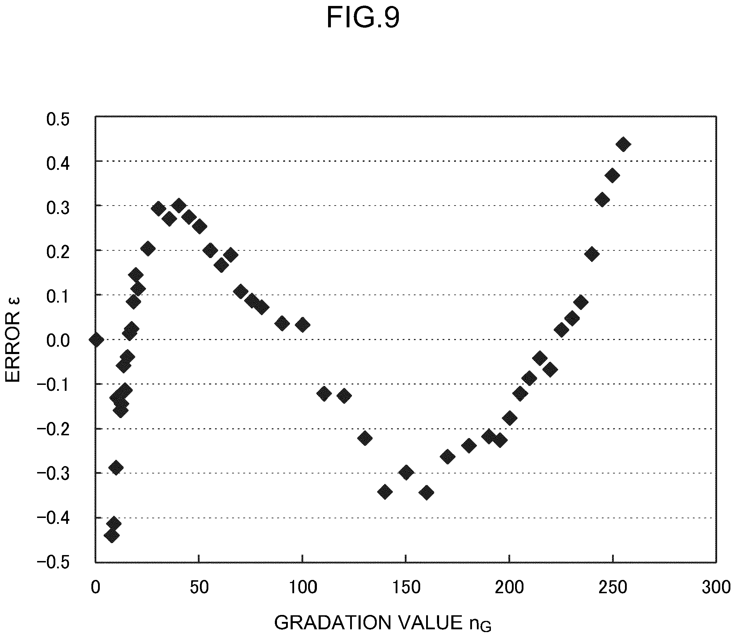

Fig. 7 is a diagram illustrating graph indication of fitting of the function represented by Formula (1) to the gradation value dependency of the chromaticity coordinates illustrated in Fig. 6. As illustrated in Fig. 7, the CPU 11 can fit the function represented by Formula (1) to the gradation value dependency of the chromaticity coordinates. In addition, Fig. 8 is a diagram illustrating graph indication of a fitting error in chromaticity coordinates when the function is fitted to the gradation value dependency of the chromaticity coordinates. As illustrated in Fig. 8, it can be seen that the chromaticity error when the function is fitted to the gradation value dependency of the chromaticity coordinates is reduced to 0.005 or less in the order of MacAdam's color discrimination ellipse. In addition, Fig. 9 is a diagram illustrating graph indication of a fitting error in luminance when the function is fitted to the gradation value dependency of the luminance. As illustrated in Fig. 9, it can be seen that the gradation value conversion error of the luminance when the function is fitted to the gradation value dependency of the chromaticity coordinates is reduced to 0.5 or less in the order of MacAdam's color discrimination ellipse. Note that the graphs illustrated in Figs. 5 to 9 indicate a green primary color of sRGB, which is one of the color space standards defined by the International Electrotechnical Commission (IEC), and the optotype presentation device 20 creates the green primary color from the red, green, and blue subpixels according to a lookup table (LUT). The function of the LUT is to adjust the luminance of the red, green, and blue subpixels to create three primary colors (red, green, and blue) of the specified chromaticity coordinates by color mixing, and the output luminance of the red, green, and blue subpixels is controlled in a set by one gradation value corresponding to one primary color.

After the predetermined function is fitted to the relationship between the tristimulus values and the gradation value in step S103, the CPU 11 subsequently receives the display target value in step S104, and determines the gradation value corresponding to the display target value using the function. The display target value is, for example, luminance and xy chromaticity coordinates in a case in which the gradation value is calculated using the function representing the tristimulus values, and is the luminance and cone stimulus values or ratio of L, M, and S in a case in which the gradation value is calculated using the function representing the LMS cone stimulus.

The method of calculating the gradation value corresponding to the display target value is not limited to the specific method. For example, the CPU 11 may calculate the gradation value corresponding to the display target value by sequential search, may first obtain a rough approximate solution and then perform sequential search in the vicinity of the approximate solution. In addition, the CPU 11 may calculate the gradation value corresponding to the display target value by applying an iterative method to the function fitted to the relationship between the tristimulus values and the gradation value.

An example of the method of calculating the gradation value corresponding to the display target value will be described. In correspondence with the fact that the gradation value is a discrete amount, the luminance and chromaticity that can be displayed by the optotype presentation device 20 are represented as grid points in the luminance space and the color space. First, the CPU 11 obtains, for example, the gradation values (decimal points) of red, green, and blue combining the chromaticity of D65, and sets a set of tristimulus values of each of red, green, and blue at this time as a reference subpixel. Subsequently, the CPU 11 obtains the gradation values of red, green, and blue corresponding to the grid points closest to desired luminance and chromaticity. When obtaining the gradation values, the CPU 11 first obtains a rough solution using linear combination in which the reference subpixel is considered as a vector, then calculates the luminance and chromaticity of the grid points (26 points in total) of plus or minus 1 of each gradation value of red, green, and blue centering on the obtained solution, and updates the initial grid point to the grid point having the closest distance to the desired luminance and chromaticity. The CPU 11 obtains the grid point having the minimum distance as a solution.

In this manner, by setting the reference subpixel first and then rereading the target xy chromaticity coordinate value of the optotype as the coefficient α of the linear combination and the target luminance value as the coefficient β of the linear combination, the pixel can be decomposed into the tristimulus values of each subpixel without explicitly including the gradation value. Subsequently, the CPU 11 can obtain the gradation value corresponding to the display target value by solving the function (Formula (1)) representing the stimulus value Y at the gradation value for each subpixel.

After the gradation value corresponding to the display target value is determined in step S104, the CPU 11 subsequently displays the optotype with the chromaticity and the luminance based on the determined gradation value on the optotype presentation device 20 in step S105.

By executing a series of processing, the information processing device 10 can fit the predetermined function to the relationship between the actual stimulus value and gradation value of the optotype presentation device 20 and determine the gradation value corresponding to the display target value using the function. By determining the gradation value corresponding to the display target value, the information processing device 10 can realize display of the optotype with accurate luminance and chromaticity on the optotype presentation device 20.

The information processing device 10 may perform the series of processing each time at the timing of starting the visual function inspection on the subject. Alternatively, for example, simple measurement of the gradation characteristics on the optotype presentation device 20 may be performed, so that the measurement may be performed again to perform fitting of the function in a case in which the variation of the gradation characteristics exceeds a predetermined threshold.

In the visual function inspection system 1 according to the present embodiment, it is assumed that the optotype presentation device 20 is the device including the red, green, and blue subpixels that represent primary colors by color mixing, such as a liquid crystal display, an organic EL display, or a CRT display. However, the optotype presentation device 20 may be a projector that presents an image on a wall surface or the like, or may present transmitted light or reflected light of an object as an optotype by irradiating the object with illumination such as LED light of a plurality of colors. Such a device represents a desired color by emitting a primary color instead of representing the primary color by mixing colors of subpixels. In a case in which such a device is used for the optotype presentation device 20, the information processing device 10 acquires, at the measurement data acquisition unit 101, the gradation characteristics which are the data set obtained by measuring the spectral radiance at the plurality of gradation values of the plurality of primary colors, and calculates, at the stimulus value calculation unit 102, the stimulus value at each gradation value based on the acquired gradation characteristics. Then, the information processing device 10 fits, at the function generation unit 103, the predetermined function related to the gradation characteristics to the relationship between the stimulus value and the gradation value.

As described above, the information processing device 10 according to the embodiment of the present disclosure can acquire the actual gradation characteristics of the optotype presentation device 20 and generate data for displaying the optotype on the optotype presentation device 20 with a desired gradation value. The information processing device 10 according to the embodiment of the present disclosure generates the data for displaying the optotype on the optotype presentation device 20 with the desired gradation value, thereby allowing the optotype presentation device 20 to present an accurate optotype and enabling accurate inspection of the visual function of the subject.

Alternatively, the information processing executed by the CPU reading software (program) in each of the embodiments may be executed by various processors other than the CPU. Examples of the processor in this case include a programmable logic device (PLD) whose circuit configuration can be changed after manufacturing such as a field-programmable gate array (FPGA), a dedicated electric circuit that is a processor having a circuit configuration exclusively designed for executing specific processing such as an application specific integrated circuit (ASIC), and the like. In addition, the information processing may be executed by one of these various processors, or may be executed by a combination of two or more processors of the same or different types (for example, a plurality of FPGAs, a combination of a CPU and an FPGA, and the like). Furthermore, the hardware structure of these various processors is, more specifically, an electric circuit in which circuit elements such as semiconductor elements are combined.

In each of the embodiments, the aspect in which the information processing program is stored (installed) in advance in the ROM or the storage has been described, but the present invention is not limited thereto. The program may be provided in a form recorded in a nontransitory recording medium such as a compact disk read only memory (CD-ROM), a digital versatile disk read only memory (DVD-ROM), or a universal serial bus (USB) memory. In addition, the program may be downloaded from an external device via a network.

The disclosure of Japanese Patent Application No. 2021-183435 filed on November 10, 2021 is incorporated herein by reference in its entirety.

### Reference Signs List

- 10: Information processing device
- 20: Optotype presentation device
- 30: Measurement device

## Claims

1. An information processing device comprising:
a measurement data acquisition unit that acquires gradation characteristics, which are a data set obtained by measuring spectral radiance of an optotype presentation device that presents an optotype to a subject, at a plurality of gradation values of a plurality of primary colors;
a stimulus value calculation unit that calculates a stimulus value at each gradation value based on the gradation characteristics; and
a function generation unit that fits a predetermined function related to the gradation characteristics to a relationship between the stimulus value and the gradation value.

2. The information processing device according to Claim 1, wherein the function fitted by the function generation unit includes a nonlinear term including a parameter for fitting and a linear term including a parameter for fitting.

3. The information processing device according to Claim 1 or 2, further comprising a gradation value calculation unit that calculates a gradation value corresponding to a display target value in the optotype presentation device based on the function.

4. The information processing device according to any one of Claims 1 to 3, wherein the stimulus value calculation unit calculates tristimulus values of an XYZ color system for each of the primary colors as the stimulus value.

5. The information processing device according to any one of Claims 1 to 3, wherein the stimulus value calculation unit calculates an LMS cone stimulus value for each of the primary colors as the stimulus value.

6. The information processing device according to any one of Claims 1 to 5, wherein the measurement data acquisition unit acquires the gradation characteristics at a timing of starting a visual function inspection on the subject.

7. The information processing device according to any one of Claims 1 to 6, wherein the measurement data acquisition unit acquires, in addition to the gradation characteristics, environmental data on an environment at a time point for measuring the gradation characteristics.

8. The information processing device according to Claim 7, wherein the function generation unit fits the predetermined function to the relationship between the stimulus value and the gradation value according to the environment data.

9. The information processing device according to any one of Claims 1 to 8, wherein the measurement data acquisition unit acquires gradation characteristics of each of red, green, and blue subpixels that represent the primary color by color mixing.

10. An information processing method wherein a computer executes processing of
acquiring gradation characteristics, which are a data set obtained by measuring spectral radiance of an optotype presentation device that presents an optotype to a subject, at a plurality of gradation values of a plurality of primary colors;
calculating a stimulus value at each gradation value based on the gradation characteristics; and
fitting a predetermined function related to the gradation characteristics to a relationship between the stimulus value and the gradation value.

11. A computer program causing a computer to execute processing of
acquiring gradation characteristics, which are a data set obtained by measuring spectral radiance of an optotype presentation device that presents an optotype to a subject, at a plurality of gradation values of a plurality of primary colors;
calculating a stimulus value at each gradation value based on the gradation characteristics; and
fitting a predetermined function related to the gradation characteristics to a relationship between the stimulus value and the gradation value.
